# EUROPÄISCHE PATENTSCHRIFT

(11) **EP 1 984 320 B1**
(45) Veröffentlichungstag und Bekanntmachung des Hinweises auf die Patenterteilung: **23.10.2013**
(21) Anmeldenummer: 07704111.9
(22) Anmeldetag: 24.01.2007
(51) Int. Cl.: C07C 209/84, C07C 211/27

(54) **VERFAHREN ZUR HERSTELLUNG VON REINEM XYLYLENDIAMIN (XDA)**
METHOD FOR PRODUCING PURE XYLYLENEDIAMINE (XDA)
PROCEDE DE PRODUCTION DE XYLYLENEDIAMINE PURE (XDA)

(30) Priorität: 01.02.2006 EP 06101145
(43) Veröffentlichungstag der Anmeldung: 29.10.2008
(73) Patentinhaber: BASF SE, 67056 Ludwigshafen (DE)
(72) Erfinder: HUGO, Randolf, 67246 Dirmstein (DE); DAHMEN, Kirsten, 68161 Mannheim (DE); HUBER, Sabine, 67117 Limburgerhof (DE)
(86) Internationale Anmeldenummer: PCT/EP2007/050679
(87) Internationale Veröffentlichungsnummer: WO 2007/088131

(56) Entgegenhaltungen:
- WO-A-2005/028417

## Beschreibung

Die vorliegende Erfindung betrifft ein Verfahren zur Herstellung eines reinen Xylylendi-amins (XDA) durch kontinuierliche Destillation des rohen Xylylendiamins in einer Kolonne.

Xylylendiamin (Bis(aminomethyl)benzol) ist ein nützlicher Ausgangsstoff, z.B. für die Synthese von Polyamiden, Epoxyhärtern oder als Zwischenstufe zur Herstellung von Isocyanaten.

Die Bezeichnung "Xylylendiamin" (XDA) umfasst die drei Isomere ortho-Xylylendiamin, meta-Xylylendiamin (MXDA) und para-Xylylendiamin.

Rohes Xylylendiamin kann beispielsweise durch Ammonoxidation von Xylol und anschließender Hydrierung des erhaltenen Phthalodinitrils hergestellt werden. Mögliche Herstellverfahren sind zum Beispiel in den nachstehend genannten Anmeldungen beschrieben.

US-A-4,482,741 (UOP Inc.) beschreibt die Hydrierung von Phthalodinitril (PDN) in Gegenwart von Ammoniak, einem spezifischen Katalysator und XDA als Lösungsmittel.

EP-A2-1 193 247 und EP-A1-1 279 661 (beide Mitsubishi Gas Chem. Comp.) betreffen ein Verfahren zur Reinigung von Isophthalodinitril (IPDN) bzw. ein Verfahren zur Herstellung von reinem XDA.

EP-A2-1 193 244 (Mitsubishi Gas Chem. Comp.) beschreibt ein Verfahren zur Herstellung von XDA durch Hydrierung von Phthalodinitril, welches in einer vorherigen Stufe durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench) und die erhaltene Quenchlösung oder -suspension der Hydrierung zugeführt wird.

Die Aufreinigung des erhaltenen rohen XDAs ist auch in folgenden Patentanmeldungen beschrieben:

JP 2002-088032 (Mitsubishi Gas Chem. Co, Inc.) beschreibt eine Methode zur Reinigung von XDA durch Einspeisung eines Inertgases zum Kondensator, wobei der Kolonnen-Kopfdruck bei 53 mbar oder weniger und die Auslasstemperatur des Kondensators bei 110°C oder weniger gehalten werden. Durch das Inertgas werden Komponenten, die leichter als XDA sieden, ausgestrippt, so dass diese nicht oder nicht vollständig kondensieren, sondern mit dem Inertgas über Kopf abgezogen werden. Eine hohe Kondensationstemperatur begünstigt den Effekt. Die Belastung der Vakuummaschine wird jedoch durch das Inertgas vergrößert, und die gestippten Komponenten können aus dem Inertgas nur schwer kondensiert werden, so dass im wesentlichen die Verbrennung in einer Muffel oder Fackel bleibt. Gemäß Anspruch 2 wird Inertgas zugegeben wie in Anspruch 1 und die Sumpftemperatur auf 180°C begrenzt. Daraus ergibt sich entweder ein großer Produktverlust über Sumpf, oder es ist ein sehr niedriges Vakuum erforderlich, was aber wegen der Inertgaszugabe schwierig und aufwendig zu erzeugen ist.

US 3,647,054 (Japan Gas Chemical) beschreibt die Reinigung des rohen Xylylendiamins durch Zugabe einer alkalischen Komponente und anschließendes Erwärmen, um Reste an nicht bzw. nicht vollständig hydriertem Phthalodinitril bzw. Cyanobenzylamin zu verseifen, um anschließend reines Xylylendiamin destillativ zu erhalten. Phthalodinitril und Cyanobenzylamin lassen sich ansonsten kaum destillativ von Xylylendiamin trennen. Die Bedingungen für die Verseifungsreaktion werden beschrieben. Über die Reindestillation wird in den Beispielen lediglich erwähnt, dass bei reduziertem Druck gearbeitet wird. Auf Abspaltungs- oder Zersetzungsprodukte, die bei höherer Temperatur aus Xylylendiamin bei der Destillation entstehen und das Produkt verunreinigen, wird in US 3,647,054 nicht eingegangen.

SU-A-322 322 vom 26.01.1968 (Galperin et al.) beschreibt die Reinigung von rohem Xylylendiamin, welches Nitrile als Begleitkomponenten enthält, indem die Begleitkomponenten bei 200°C in Gegenwart von Lauge oder Ammoniak hydrolysiert werden. Die Reindestillation ist nicht näher beschrieben. Auf die Problematik der Produktzersetzung oder Abspaltung von Ammoniak bei höherer Temperatur wird nicht eingegangen.

JP-B-700 14 777 (Japan Gas Chem. Co) beschreibt die Reinigung von Xylylendiamin durch Zugabe von 0,5 bis 5 Gew.-% einer alkalischen Substanz zum rohen Xylylendiamin, bezogen auf nicht abreagiertes Phthalonitril. Das rohe Xylylendiamin wurde durch Hydrierung von Phthalonitril in homogener flüssiger Phase aus flüssigem Ammoniak und organischem Lösungsmittel erhalten. Anschließend wird die Mischung destilliert.

JP-A-2003 026638 (Mitsubishi Gas Chem. Co, Inc.) beschreibt ein aufwendiges Extraktionsverfahren mit Wasser und aromatischen oder gesättigten Kohlenwasserstoffen zur XDA-Aufreinigung. Dazu wird Phthalonitril durch Ammonoxidation von Xylol hergestellt, das Reaktionsgas mit Lösungsmittel gequencht, mit Ammoniak versetzt und hydriert. Nachdem Ammoniak und Lösungsmittel abgetrennt sind, erhält man rohes Xylylendiamin. Es werden Wasser und mindestens ein aromatisches oder gesättigtes Lösungsmittel zugegeben. Nach Phasentrennung wird reines Xylylendiamin mit der wässrigen Phase erhalten, von welcher reines Xylylendiamin durch Destillation gewonnen wird. Um reines XDA zu erhalten, werden in den oben genannten Publikationen teilweise aufwendige Verfahren beschrieben, mit denen XDA z.B. extraktiv gereinigt wird, oder es werden Hilfsstoffe hinzugefügt, welche die geforderte hohe Reinheit des XDA gewährleisten sollen. Durch die Hilfsstoffzugabe soll entsprechendes Nitril, welches durch unvollständige Reaktion nach der Hydrierung noch als Verunreinigung im rohen Xylylendiamin enthalten ist, verseift werden.

Die beschriebenen Verfahren sind apparativ und durch die Handhabung großer Lösungsmittelströme bzw. zusätzlicher Komponenten aufwendig.

Die beiden deutschen Patentanmeldungen Nr. 102005003315.6 vom 24.01.05 und Nr. 102005008929.1 vom 24.02.05 (beide BASF AG) betreffen die PDN-Niederdruckhydrierung in Gegenwart von Raney-Katalysatoren.

WO-A-05/028417, WO-A-05/026102, WO-A-05/026103, WO-A-05/026104, WO-A-05/026100, WO-A-05/026101, WO-A-05/026098, WO-A-05/026099 und die beiden deutschen Patentanmeldungen Nr. 102005036222.2 vom 02.08.05 und Nr. 102005045806.8 vom 24.09.05 (alle BASF AG) betreffen jeweils Verfahren zur Herstellung von XDA. In all diesen Verfahren wird rohes Xylylendiamin erhalten, das anschließend weiter aufgereinigt werden muss.

Z.B. lehrt WO-A-05/028417 auf Seite 8, dass die Abtrennung leichter- und schwerersiedender Nebenprodukte auch in einer Seitenabzugs- oder Trennwandkolonne erfolgen kann, wobei reines Xylylendiamin über einen flüssigen oder gasförmigen Seitenabzug gewonnen wird. Aufgrund von Reaktionen im Destillationssumpf von Roh-XDA bei hohen Temperaturen lässt sich auf diese Weise XDA nur dann in guter Ausbeute gewinnen, wenn die Destillation bei sehr niedrigem Druck erfolgt, um die Sumpftemperatur zu begrenzen. Dieses ist apparativ relativ aufwendig. Um reines XDA im Seitenabzug zu gewinnen, muss bei großtechnisch leichter zu realisierendem Vakuum ein Produktverlust über Sumpf in Kauf genommen werden. Bei Erhöhung der Ausbeute führt die ansteigende Sumpftemperatur zu Produktzersetzung, wobei die Zersetzungsprodukte, wie z.B. Methylbenzylamin, das im Seitenabzug entnommene XDA verunreinigen. Man kann also entweder reines XDA bei relativ niedrigerer Ausbeute oder verunreinigtes XDA bei höherer Ausbeute gewinnen.

Der vorliegenden Erfindung lag die Aufgabe zugrunde, ein verbessertes wirtschaftliches Verfahren zur destillativen Reinigung von rohem Xylylendiamin, insbesondere meta-Xylylendiamin (MXDA), aufzufinden, bei welchem Xylylendiamin in hoher Reinheit und gleichzeitig in hoher Ausbeute gewonnen werden kann.

Mit rohem Xylylendiamin ist in diesem Dokument insbesondere der Reaktionsaustrag aus der Hydrierung des entsprechenden Phthalodinitrils (PDNs) gemeint, von welchem ggf. Ammoniak ganz oder weitgehend sowie ein eventuell in der Hydrierung eingesetztes Lösemittel ganz oder weitgehend abgetrennt ist und der noch höhersiedende Verunreinigungen sowie leichtersiedende Komponenten, darunter ggf. auch ein Lösungsmittel, enthält.

Bei der destillativen Reinigung von XDA besteht u.a. das Problem, dass durch Produktzersetzung Ammoniak und/oder leichtsiedende Komponenten wie z.B. Methyl-benzylamin freigesetzt werden, welche das Produkt verunreinigen.

Überraschenderweise konnte experimentell gezeigt werden, dass reines Xylylendiamin durch Destillation von rohem Xylylendiamin erhalten werden kann, wobei das Xylylen-diamin mittels einer Seitenabzugskolone als Seitenabzug erhalten und die Destillation bei technisch einfach zu realisierendem Vakuum betrieben wird.

Demgemäß wurde ein Verfahren zur Herstellung eines reinen Xylylendiamins (XDA) durch kontinuierliche Destillation des rohen Xylylendiamins in einer Kolonne gefunden, welches dadurch gekennzeichnet ist, dass eine Destillationskolonne mit Seitenabzug eingesetzt und das XDA im Seitenabzug abgezogen wird, wobei die Destillationskolonne im Vakuum betrieben wird und die Sumpftemperatur 185°C oder weniger beträgt, und das Sumpfprodukt der Destillationskolonne in einer zusätzlichen Eindampfstufe weiter eingedampft und das Kondensat dieser Eindampfstufe in die Destillationskolonne mit Seitenabzug zurückgefahren wird.

Beim erfindungsgemäßen Verfahren sind zusätzliche Verfahrensschritte, wie z.B. Extraktionsschritte, nicht unbedingt erforderlich.

In einer bevorzugten Ausführungsform kann auf die Zugabe von Hilfsstoffen [wie z.B. KOH, NaOH, Na- bzw. K-Phthalimid oder sonstige alkalische Komponenten (Basen), jeweils in reiner Form oder als Lösung, sowie reduzierende Komponenten wie NaBH₄ oder LiAlH₄, sowie Kombinationen einer Base mit einer reduzierenden Komponente, sowie H₃PO₃] verzichtet werden, sofern diese nicht zur Verseifung von nicht vollständig hydrierten Nitrilen eingesetzt werden. Die Zugabe von Hilfsstoffen zur Verseifung von Nitrilen (wie z.B. Phthalodinitril bzw. Cyanobenzylamin) kann bei unvollständiger Reaktion in der vorhergehenden Hydrierstufe nützlich sein. Die in der vorliegenden Erfindung beschriebene Zersetzung des Amins bzw. die weitgehende Unterdrückung der Zersetzung bzw. die Gewinnung von Rein-XDA in guter Ausbeute ist bei hinreichender Nitrilfreiheit des Rohproduktes von Hilfsstoffzugaben gemäß dem oben geschilderten Stand der Technik unabhängig.

Beispielsweise wird bei der Destillation von meta-Xylylendiamin Ammoniak und meta-Methylbenzylamin freigesetzt, welches einen niedrigeren Siedepunkt als meta-Xylylendiamin aufweist. Es werden bei der Destillation ständig neue Leichtsieder gebildet, welche als Verunreinigungen im destillierten Xylylendiamin auftauchen. Durch Begrenzen der Sumpftemperatur in der Destillation auf ≤ 185°C, insbesondere auf ≤ 180°C, z.B. auf eine Temperatur im Bereich von 160 bis 185°C, besonders 170 bis 180°C, lässt sich die Produktzersetzung auf ein tolerierbares Maß einschränken.

Dies wird erreicht durch Anlegen eines hinreichend niedrigen Vakuums und/oder durch Begrenzung des Sumpfeindampfgrades und/oder Minimieren der Verweilzeit im Sumpf. Alle Alternativen sind zunächst noch unbefriedigend, da sie durch aufwendige Vakuumerzeugung und vergrößerte Destillationskolonnen apparativ teuer sind oder zu einem hohen Produktverlust und entsprechend verkleinerter Wertproduktausbeute führen. Die Verweilzeit im Sumpf kann vorteilhaft auch durch konstruktive Maßnahmen, insbesondere Verkleinerung des Kolonnendurchmessers im Sumpfbereich (gegenüber dem darüber befindlichen Kolonnenkörper), z.B. um 25 bis 60 %, verkürzt werden. Der Durchmesser wird dabei so gewählt, dass der Sumpfstand und dessen mögliche Schwankungsbreite bei reduziertem Sumpfvolumen zur Regelung des Sumpfaustrages und zum Betrieb einer ggf. verwendeten Sumpfumlaufpumpe ausreichend bemessen sind. Diese Dimensionierung kann von einem entsprechenden Fachmann durchgeführt werden.

Ein Vorteil der Seitenabzugskolonne gegenüber einer herkömmlichen Destillationskolonne ist, dass hierdurch ein Großteil der im Sumpf gebildeten Leichtsieder über Kopf ausgetragen werden und somit eine höhere Reinheit an XDA erzielt werden kann. Somit können höhere Sumpftemperaturen erlaubt werden, als mit einer "normalen" Kolonne, bei der das Produkt am Kolonnenkopf zusammen mit den gebildeten Leichtsiedern anfällt.

Die Destillationskolonne mit Seitenabzug wird im Vakuum betrieben. Der Druck im Kolonnensumpf (= Kolonnenkopfdruck plus Druckverlust über die Kolonne) beträgt vorteilhaft ≤ 200 mbar, besonders ≤ 100 mbar, insbesondere ≤ 50 mbar, z.B. im Bereich von 30 bis 50 mbar.

Vorteilhaft werden Kolonneneinbauten verwendet, welche im Betrieb nur einen geringen Druckverlust aufweisen, um einerseits den Aufwand zur Vakuumerzeugung klein zu halten und andererseits das Sumpfprodukt möglichst wenig thermisch zu beanspruchen. Entscheidend für die thermische Belastung des Sumpfproduktes ist die Sumpftemperatur, welche sich aus Sumpfkonzentration und Druck im Kolonnensumpf ergibt.

Das im erfindungsgemäßen Verfahren eingesetzte rohe XDA weist z.B. eine Reinheit im Bereich von 85 bis 99,7 Gew.-%, insbesondere 90 bis 99,5 Gew.-%, auf. Der Anteil an Leichtersiedern, wie z.B. Benzylamin und entsprechendem Methyl-benzylamin (d.h. 2-, 3- bzw. 4-Methyl-benzylamin) liegt bevorzugt im Bereich von 0,01 bis 2 Gew.-% 2-, 3- bzw. 4-Methyl-benzylamin) liegt bevorzugt im Bereich von 0,01 bis 2 Gew.-% (jeweils bezogen auf die Rohware ohne Ammoniak und Lösungsmittel), der Anteil an Höhersiedern, wie z.B. Amide, Amidine, Bis-XDA und höhere Oligomere, liegt z.B. im Bereich von 0,3 bis 13 Gew.-%, besonders im Bereich von 0,5 bis 9 Gew.-%.

Unter 'Höhersieder' sind Komponenten zu verstehen, die unter gleichen Bedingungen einen höheren Siedepunkt als das jeweilige Xylylendiamin aufweisen.

Bei den Höhersiedern handelt es sich z.B. um Amide, Amidine, Bis-XDA (XDA-Dimere), und weitere Oligomere, z.B. gemäß der folgenden Formeln:
Amide: z.B. 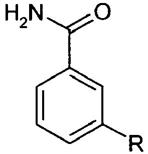 R = -CH₂NH₂, -CN, -CONH₂, -CH₂NHCH₂-Aryl, -C(NH)NCH₂-Aryl, -CHNCH₂-Aryl
Amidine: z.B. 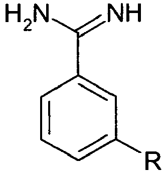 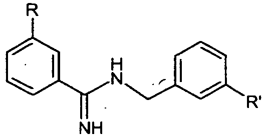 R, R' (unabhängig voneinander) = -CH₂NH₂, -CN, -CONH₂, -CH₂NHCH₂-Aryl, -C(NH)NCH₂-Aryl, -CHNCH₂-Aryl
Bis-XDA: z.B. Bis-MXDA 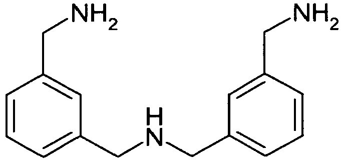
Sonstige Oligomere: z.B. 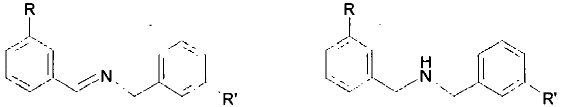 R, R' (unabhängig voneinander) = -CH₂NH₂, -CN, -CONH₂, -CH₂NHCH₂-Aryl, -C(NH)NCH₂-Aryl, -CHNCH₂-Aryl

Der Sumpfaustrag wird dann einer weiteren Verdampferstufe zugeführt, bei der bevorzugt ein gleiches oder niedrigeres Vakuum [oder aber kürzere Verweilzeit, ggf. auch besseres Vakuum und kürzere Verweilzeit] als am Kopf der Destillationskolonne mit Seitenabzug angelegt wird, und in der der Feedstrom soweit als möglich eingedampft wird.

Insbesondere ist es auch möglich, das Vakuum für die Destillationskolonne mit Seitenabzug und die nachgeschaltete Eindampfstufe mit der gleichen Vakuummaschine zu erzeugen, wobei sich in der nachgeschalteten Eindampfstufe ein geringerer Druck als im Sumpf der Destillationskolonne ergibt, da in der Kolonne mit Seitenabzug der Druckverlust auf Grund der Kolonneneinbauten größer ist. Bei Beachtung der oben genannten Punkte ist die Produktzersetzung eingeschränkt. Da das in der Nachstufe erhaltene XDA, erneut über die Seitenabzugskolonne gefahren wird, ist die Zersetzung in der nachgeschalteten Eindampfstufe für die Qualität des XDAs nicht ausschlaggebend, so dass hier die Stoffverluste durch Zersetzung und die anfallende Rückstandsmenge gegeneinander abgewogen werden können.

Die Brüden der nachgeschalteten Eindampfstufe werden bevorzugt kondensiert und dann in flüssiger Form der Destillationskolonne mit Seitenabzug wieder zugeführt. Brüdenkompression und dampfförmige Zuführung zur Destillationskolonne ist in einer alternativen Variante ebenfalls möglich. In jedem Fall erfolgt bei einer Kolonne mit dampfförmigem Seitenabzug die Zuführung oberhalb des Seitenabzuges.

Bei einer Kolonne mit flüssigem Seitenabzug liegt der Zulauf des Roh-XDA sowie der Zulauf des zurückgeführten XDAs aus der Eindampfstufe bevorzugt jeweils unterhalb des Seitenabzuges.

Der Seitenabzug für reines Xylylendiamin ist bevorzugt als dampfförmiger Seitenabzug im Abtriebsteil der Seitenabzugskolonne angeordnet.

In einer besonderen Ausführungsform kann als Destillationskolonne mit Seitenabzug vorteilhaft eine Trennwanddestillationskolonne mit Seitenabzug zum Einsatz kommen. Hierbei kann eine Verunreinigung des Produktes Xylylendiamin durch Zersetzung im Sumpf noch weiter reduziert werden.

In diesem Fall wird bevorzugt ein flüssiger Seitenabzug im Bereich der Trennwand verwendet. Die Zugabe der kondensierten oder komprimierten Brüden der nachgeschalteten Eindampfstufe erfolgt bevorzugt auf der (der Seitenabzugsseite gegenüber liegenden) Zulaufseite im Bereich der Trennwand.

Im Falle einer Trennwandkolonne kann die Zugabe der kondensierten oder komprimierten Brüden der nachgeschalteten Eindampfstufe auch auf gleicher Höhe oder sogar unterhalb des Seitenabzuges angeordnet sein, in jedem Fall jedoch bevorzugt im Bereich der Trennwand.

Bevorzugt findet das erfindungsgemäße Verfahren Anwendung zur Herstellung von reinem meta-Xylylendiamin (MXDA), ausgehend von rohem meta-Xylylendiamin, welches durch Hydrierung von Isophthalodinitril (IPDN) erhalten wurde, welches wiederum insbesondere in einer vorherigen Stufe durch Ammonoxidation von meta-Xylol synthetisiert wurde.

Das erfindungsgemäße Verfahren liefert bevorzugt ein reines XDA mit einer Reinheit von ≥ 99,9 Gew.-%, insbesondere ≥ 99,94 Gew.-%.

Das mit dem erfindungsgemäßen Verfahren hergestellte reine XDA weist bevorzugt ein Restgehalt an entsprechendem Methyl-benzylamin (d.h. 2-, 3- bzw. 4-Methyl-benzylamin) von ≤ 500 ppm, besonders ≤ 300 ppm, ganz besonders ≤ 250 ppm, speziell ≤ 150 ppm, z.B. im Bereich von 5 bis 140 ppm, auf.

Das erfindungsgemäße Verfahren liefert bevorzugt ein reines XDA mit einer Destillationsausbeute von ≥ 95 %, insbesondere von ≥ 97 %, jeweils bezogen auf die Menge an Rein-XDA im eingesetzten Roh-XDA.

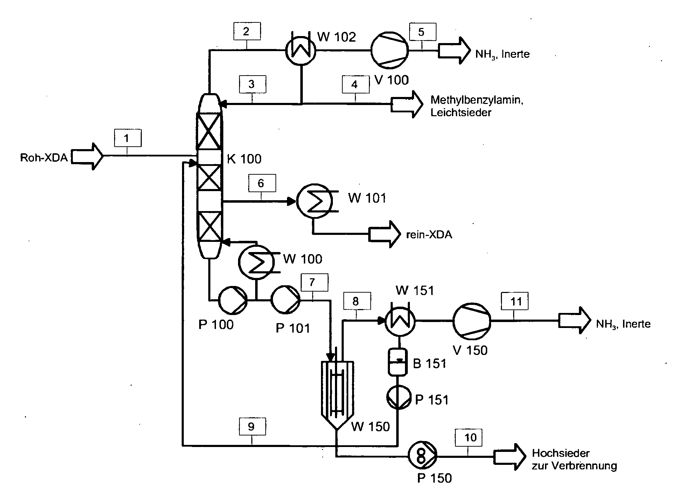

Das Verfahren wird im folgenden in einer bevorzugten Variante anhand der Abbildung 1 (in der Anlage) näher erläutert.

Rohes XDA, welches von NH₃ und ggf. Lösungsmitteln weitgehend befreit ist, wird in den mittleren Bereich einer im Vakuum betriebenen Seitenabzugskolonne zugefahren (Strom [1]) und dort in Leichtsieder (Strom [4]), rein-XDA (Strom [6]) und Hochsieder (Strom [7]) getrennt. Im Abtriebsteil der Kolonne befindet sich ein dampfförmiger Seitenabzug, über den reines XDA dampfförmig abgezogen (Strom [6]) und anschließend kondensiert wird. Die Sumpfheizung erfolgt bevorzugt über einen dampfbeheizten Wärmeübertrager (W 100). Die Sumpftemperatur beträgt bevorzugt nicht mehr als 180°C, um die Bildung von Ammoniak und Methylbenzylamin aus Xylylendiamin in Grenzen zu halten. Das Kopfprodukt wird im Wärmeübertrager W 102 kondensiert und teilweise als Rücklauf zur Kolonne zurückgeführt (Strom [3]). Es enthält die leichtsiedenden Komponenten, insbesondere Methylbenzylamin, aber auch Benzylamin, ggf. im Roh-XDA enthaltenes Lösungsmittel oder Reste davon. Ammoniak wird zum Großteil zusammen mit Inertgasen über die Vakuummaschine abgezogen (Strom [5]). Das Sumpfprodukt (Strom [7]) wird einer weiteren Eindampfstufe zugeführt (W 150). Dies kann z.B. ein Dünnschicht- oder Fallfilmverdampfer oder ein sonst wie geeigneter Wärmeübertrager sein. Die Temperatur in W 150 kann höher sein als in W 100. Bevorzugt ist die Temperatur aber in etwa gleich, wie die Temperatur in W 100, aber es wird bei kleinerem Druck nachverdampft. Aufgrund der geringeren Verweilzeit (VWZ) wird beim Aufkonzentrieren die Zersetzung so gering wie möglich gehalten. Das Kopfprodukt des Verdampfers (Strom [8]) wird in W 151 kondensiert. Inerte und NH₃ werden über die Vakuummaschine abgezogen. Der eingeengte Sumpf (Strom [10]) enthält nur noch wenig Xylylendiamin und wird aus dem Verfahren ausgeschleust. Das Kondensat (Strom [9]) wird zur Kolonne K 100 zurückgeführt, und zwar oberhalb des Seitenabzugs. Der Zulauf kann oberhalb unterhalb oder auf gleicher Höhe wie der Zulauf des Roh-XDAs sein. Bevorzugt ist er auf gleicher Höhe. Wenn das Roh-XDA noch größere Mengen an Lösungsmittel enthält, kann es sinnvoll sein, den Zulauf von Strom [9] unterhalb des Hauptzulaufs anzuordnen, in jedem Fall aber oberhalb des Seitenabzugs.

Falls das Roh-XDA nur geringe Mengen an leichtsiedenden Komponenten enthält, kann auch eine Kolonne mit flüssigem Seitenabzug im Verstärkungsteil zur Reindestillation des XDA verwendet werden. Das Verfahren ist dann analog zum oben beschriebenen Verfahren mit dampfförmigem Seitenabzug, jedoch befinden sich die Zuläufe für Roh-XDA sowie des zurückgeführten Kondensats der zusätzlichen Eindampfstufe unterhalb des flüssigen Seitenabzuges.

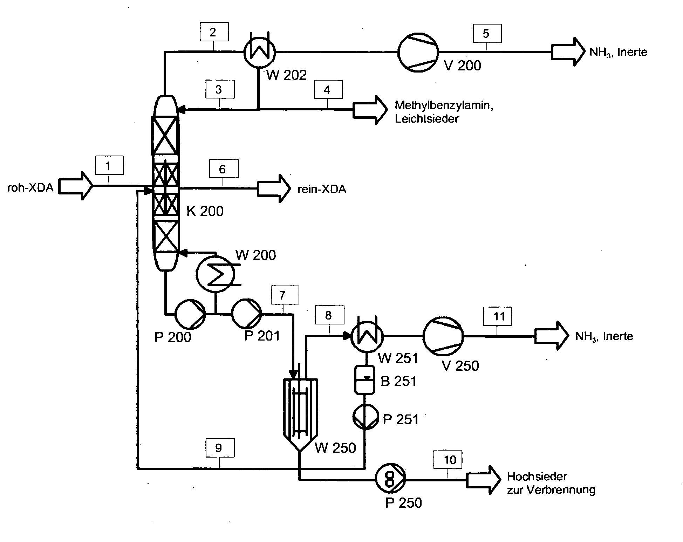

Wird statt einer Seitenabzugs- eine Trennwandkolonne verwendet, so ändert sich das Verfahren in einer bevorzugten Variante nur leicht: Abbildung 2 (in der Anlage). Die Trennwand (das Trennblech) teilt die Kolonne in einen Zulaufteil (Seite, auf der der roh-XDA-Zulaufstrom zur Kolonne zugefahren wird) und einen Entnahmeteil (Seite, auf der der rein-XDA-Seitenabzug aus der Kolonne herausgefahren wird).

Der Zulauf des Roh-XDAs befindet sich im Bereich der Kolonnenmitte im Bereich der Trennwand. Der Seitenabzug von rein-XDA kann dampfförmig sein, jedoch wird bevorzugt ein flüssiger Seitenabzug verwendet (Strom [6]). Der Seitenabzug befindet sich auf der Entnahmeseite (in Abbildung 2 der Kolonnenteil rechts von der Trennwand) im Bereich der Trennwand. Der Rückführstrom aus der Nachverdampfung (Strom [9]) wird im Bereich der Trennwand auf der Zulaufseite (in Abbildung 2 der Kolonnenteil links von der Trennwand) zurückgeführt, bevorzugt unterhalb oder auf gleicher Höhe wie der Zulauf des roh-XDA-Stromes. Ansonsten gelten die gleichen Randbedingungen und Apparateverschaltungen wie bei Verwendung einer Seitenabzugskolonne analog Abbildung 1.

Alle ppm-Angaben in diesem Dokument beziehen sich auf das Gewicht.

### Beispiele

### Beispiel 1

Roh-MXDA wurde in zwei hintereinander geschalteten Kolonnen abdestilliert. In der ersten Kolonne wurden über Kopf Leichtsieder abgetrennt (jeweils kleinere Mengen an Wasser, Methylbenzylamin und Ammoniak). Über Sumpf wurde rohes MXDA abgezogen und der zweiten Kolonne zugefahren. In der zweiten Kolonne wurde MXDA über einen dampfförmigen Seitenabzug im Abtriebsteil abgezogen, während über Kopf neu gebildete Leichtsieder (Methylbenzylamin und Ammoniak) abgetrennt wurden. In der zweiten Kolonne betrug die Sumpftemperatur 180°C. Die Reinheit des abgezogenen MXDA erreichte 99,95 % (GC-Flächen-%). An Nebenkomponenten waren neben Spuren an Wasser und methyliertem MXDA insbesondere 80 ppm meta-Methylbenzylamin (Mittelwert über die gesamte Kampagne) vorhanden. Im Sumpf der ersten Kolonne konnten hingegen nur weniger als 10 ppm meta-Methylbenzylamin nachgewiesen werden, d.h. meta-Methylbenzylamin wurde in der zweiten Kolonne neu gebildet. Im Sumpf der zweiten Kolonne waren im Durchschnitt ca. 50 Gew.-% MXDA enthalten. Die Destillationsausbeute betrug 87 %.

Das Beispiel zeigt, dass in einer Seitenabzugskolonne nicht ohne weiteres gleichzeitig eine bestimmte hohe Reinheit und eine bestimmte hohe Ausbeute zu realisieren sind.

### Vergleichsbeispiel 2

Roh-MXDA wurde in einer diskontinuierlichen Destillationsapparatur destilliert. Bei 40 mbar Kopfdruck enthielt die letzte Charge, welche bei einer Sumpftemperatur zwischen 179°C und 200°C erhalten wurde, 0,94 Gew.-% meta-Methylbenzylamin. Das verbleibende Sumpfprodukt enthielt noch 59,4 Gew.-% MXDA. Das Sumpfprodukt wurde im Dünnschichtverdampfer weiter aufkonzentriert und war selbst bei 10 % Gew. % MXDA-Anteil noch gut fließfähig. Das Kopfprodukt enthielt wegen der hohen Sumpftemeratur ca. 1 Gew.-% meta-Methylbenzylamin. Nach erneuter Destilla-tion wurde daraus spezifikationsgerechtes (> 99,9 Gew.-% MXDA sowie meta-Methyl-benzylamin -100 ppm) MXDA erhalten.

Das Beispiel zeigt, wie sehr die Produktzersetzung bei höherer Temperatur ansteigt. Ferner zeigt das Beispiel, dass das Sumpfprodukt sehr stark eingeengt werden kann und dann immer noch technisch gut handhabbar ist. Das Beispiel zeigt weiter, dass das Kopfprodukt der Sumpfausquetschung mit hohem Gehalt an Methylbenzylamin erneut destilliert werden kann und daraus MXDA in hoher Reinheit gewonnen werden kann. Somit zeigt das Beispiel die erfindungsgemäße Aufkonzentrierung des Sumpfes, das Auffangen des (nicht hinreichend reinen) Destillats und die Rückführung zur Destillation, wodurch die Destillationsausbeute gesteigert wird. Es geht nur sehr wenig MXDA mit dem aufkonzentrierten Sumpfprodukt sowie durch Zersetzung verloren.

## Patentansprüche

1. Verfahren zur Herstellung eines reinen Xylylendiamins (XDA) durch kontinuierliche Destillation des rohen Xylylendiamins in einer Kolonne, **dadurch gekennzeichnet, dass** eine Destillationskolonne mit Seitenabzug eingesetzt und das XDA im Seitenabzug abgezogen wird, wobei die Destillationskolonne im Vakuum betrieben wird und die Sumpftemperatur 185°C oder weniger beträgt, und das Sumpfprodukt der Destillationskolonne in einer zusätzlichen Eindampfstufe weiter eingedampft und das Kondensat dieser Eindampfstufe in die Destillationskolonne mit Seitenabzug zurückgefahren wird.

2. Verfahren nach Anspruch 1, **dadurch gekennzeichnet, dass** das XDA über einen dampfförmigen Seitenabzug im Abtriebsteil der Destillationskolonne abgezogen wird.

3. Verfahren nach einem der vorhergehenden Ansprüche, **dadurch gekennzeichnet, dass** das Kondensat der zusätzlichen Eindampfstufe in die Destillationskolonne zurückgefahren wird, und zwar oberhalb des Seitenabzuges.

4. Verfahren nach einem der vorhergehenden Ansprüche, **dadurch gekennzeichnet, dass** als Destillationskolonne mit Seitenabzug eine Trennwanddestillationskolonne mit Seitenabzug eingesetzt wird.

5. Verfahren nach dem vorhergehenden Anspruch, **dadurch gekennzeichnet, dass** das XDA über einen flüssigen Seitenabzug im Bereich der Trennwand abgezogen wird.

6. Verfahren nach einem der beiden vorhergehenden Ansprüche, **dadurch gekennzeichnet, dass** das Kondensat der zusätzlichen Eindampfstufe in die Destillationskolonne zurückgefahren wird, und zwar im Bereich der Trennwand auf der Zulaufseite.

7. Verfahren nach einem der vorhergehenden Ansprüche, **dadurch gekennzeichnet, dass** das eingesetzte rohe XDA rohes meta-Xylylendiamin (MXDA) ist.

8. Verfahren nach einem der vorhergehenden Ansprüche, **dadurch gekennzeichnet, dass** das eingesetzte rohe XDA eine Reinheit im Bereich von 85 bis 99,7 Gew.-% aufweist.

9. Verfahren nach einem der vorhergehenden Ansprüche, **dadurch gekennzeichnet, dass** die Destillationskolonne mit Seitenabzug bei einem Druck im Kolonnensumpf von 200 mbar oder niedriger betrieben wird.

10. Verfahren nach einem der Ansprüche 1 bis 8, **dadurch gekennzeichnet, dass** die Destillationskolonne mit Seitenabzug bei einem Druck im Kolonnensumpf von 100 mbar oder niedriger betrieben wird.

11. Verfahren nach einem der Ansprüche 1 bis 8, **dadurch gekennzeichnet, dass** die Destillationskolonne mit Seitenabzug bei einem Druck im Kolonnensumpf von 50 mbar oder niedriger betrieben wird.

12. Verfahren nach einem der vorhergehenden Ansprüche, **dadurch gekennzeichnet, dass** die zusätzliche Eindampfstufe beim gleichem oder einem niedrigeren Druck wie/als der Kopfdruck der Destillationskolonne mit Seitenabzug betrieben wird.

13. Verfahren nach einem der vorhergehenden Ansprüche zur Herstellung von XDA mit einer Reinheit von ≥ 99,9 Gew.-%.

14. Verfahren nach einem der Ansprüche 1 bis 6 oder 8 bis 13 zur Herstellung von ortho-XDA mit einem Restgehalt an 2-Methyl-benzylamin von ≤ 500 Gew.-ppm.

15. Verfahren nach einem der Ansprüche 1 bis 13 zur Herstellung von meta-XDA mit einem Restgehalt an 3-Methyl-benzylamin von ≤ 500 Gew.-ppm.

16. Verfahren nach einem der Ansprüche 1 bis 6 oder 8 bis 13 zur Herstellung von para-XDA mit einem Restgehalt an 4-Methyl-benzylamin von ≤ 500 Gew.-ppm.

17. Verfahren nach einem der vorhergehenden Ansprüche, wobei der Durchmesser der Destillationskolonne mit Seitenabzug im Sumpfbereich gegenüber dem darüber befindlichen Kolonnenkörper reduziert ist.

## Claims

1. A process for preparing pure xylylenediamine (XDA) by continuously distilling crude xylylenediamine in a column, which comprises using a distillation column with side draw and drawing off the XDA in the side draw, wherein the distillation column is operated under reduced pressure and the bottom temperature is 185°C or less, and the bottom product of the distillation column is evaporatively concentrated in an additional evaporation stage and the condensate of this evaporation stage is returned into the distillation column with side draw.

2. The process according to claim 1, wherein the XDA is drawn off via a vaporous side draw stream in the stripping section of the distillation column.

3. The process according to any of the preceding claims, wherein the condensate of the additional evaporation stage is returned into the distillation column above the side draw.

4. The process according to any of the preceding claims, wherein the distillation column with side draw used is a dividing wall distillation column with side draw.

5. The process according to the preceding claim, wherein the XDA is drawn off via a liquid side draw stream in the region of the dividing wall.

6. The process according to either of the preceding claims, wherein the condensate of the additional evaporation stage is returned into the distillation column in the region of the dividing wall on the feed side.

7. The process according to any of the preceding claims, wherein the crude XDA used is crude meta-xylylenediamine (MXDA).

8. The process according to any of the preceding claims, wherein the crude XDA used has a purity in the range from 85 to 99.7% by weight.

9. The process according to any of the preceding claims, wherein the distillation column with side draw is operated under reduced pressure, at a pressure in the column bottom of 200 mbar or lower.

10. The process according to any of claims 1 to 8, wherein the distillation column with side draw is operated at a pressure in the column bottom of 100 mbar or lower.

11. The process according to any of claims 1 to 8, wherein the distillation column with side draw is operated at a pressure in the column bottom of 50 mbar or lower.

12. The process according to any of the preceding claims, wherein the additional evaporation stage is operated at the same pressure as or a lower pressure than the top pressure of the distillation column with side draw.

13. The process according to any of the preceding claims for preparing XDA having a purity of ≥ 99.9% by weight.

14. The process according to any of claims 1 to 6 or 8 to 13 for preparing ortho-XDA having a residual content of 2-methylbenzylamine of ≤ 500 ppm by weight.

15. The process according to any of claims 1 to 13 for preparing meta-XDA with a residual content of 3-methylbenzylamine of ≤ 500 ppm by weight.

16. The process according to any of claims 1 to 6 or 8 to 13 for preparing para-XDA having a residual content of 4-methylbenzylamine of ≤ 500 ppm by weight.

17. The process according to any of the preceding claims, the diameter of the distillation column with side draw being reduced in the bottom region compared to the column part disposed above it.

## Revendications

1. Procédé pour la préparation d'une xylylènediamine (XDA) par distillation continue de la xylylènediamine brute dans une colonne, **caractérisé en ce qu'**on utilise une colonne de distillation équipée d'un soutirage latéral et la XDA est soutirée par le soutirage latéral, la colonne de distillation étant exploitée sous vide et la température du fond étant de 185°C ou moins, et le produit du fond de la colonne de distillation est évaporé davantage dans une étape d'évaporation supplémentaire et le condensat de cette étape d'évaporation est recyclé dans la colonne de distillation équipée d'un soutirage latéral.

2. Procédé selon la revendication 1, **caractérisé en ce que** la XDA est soutirée via un soutirage latéral sous forme vapeur dans la partie d'épuisement de la colonne de distillation.

3. Procédé selon l'une quelconque des revendications précédentes, **caractérisé en ce que** le condensat de l'étape d'évaporation supplémentaire est recyclé dans la colonne de distillation et ce au-dessus du soutirage latéral.

4. Procédé selon l'une quelconque des revendications précédentes, **caractérisé en ce qu'**on utilise, comme colonne de distillation équipée d'un soutirage latéral, une colonne de distillation à paroi de séparation équipée d'un soutirage latéral.

5. Procédé selon la revendication précédente, **caractérisé en ce que** la XDA est soutirée via un soutirage latéral liquide dans la zone de la paroi latérale.

6. Procédé selon l'une quelconque des deux revendications précédentes, **caractérisé en ce que** le condensat de l'étape d'évaporation supplémentaire est recyclé dans la colonne de distillation et ce dans la zone de la paroi de séparation, côté alimentation.

7. Procédé selon l'une quelconque des revendications précédentes, **caractérisé en ce que** la XDA brute utilisée est la méta-xylylènediamine (MXDA) brute.

8. Procédé selon l'une quelconque des revendications précédentes, **caractérisé en ce que** la XDA brute utilisée présente une pureté dans la plage de 85 à 99,7% en poids.

9. Procédé selon l'une quelconque des revendications précédentes, **caractérisé en ce que** la colonne de distillation équipée d'un soutirage latéral est exploitée à une pression dans le fond de la colonne de 200 mbars ou moins.

10. Procédé selon l'une quelconque des revendications 1 à 8, **caractérisé en ce que** la colonne de distillation équipée d'un soutirage latéral est exploitée à une pression dans le fond de la colonne de 100 mbars ou moins.

11. Procédé selon l'une quelconque des revendications 1 à 8, **caractérisé en ce que** la colonne de distillation équipée d'un soutirage latéral est exploitée à une pression dans le fond de la colonne de 50 mbars ou moins.

12. Procédé selon l'une quelconque des revendications précédentes, **caractérisé en ce que** l'étape d'évaporation supplémentaire est exploitée à une pression identique à la pression de tête ou plus basse que celle-ci de la colonne de distillation équipée d'un soutirage latéral.

13. Procédé selon l'une quelconque des revendications précédentes pour la préparation de XDA présentant une pureté ≥ 99,9% en poids.

14. Procédé selon l'une quelconque des revendications 1 à 6 ou 8 à 13 pour la préparation d'ortho-XDA présentant une teneur résiduelle en 2-méthylbenzylamine ≤ 500 ppm en poids.

15. Procédé selon l'une quelconque des revendications 1 à 13 pour la préparation de méta-XDA présentant une teneur résiduelle en 3-méthylbenzylamine ≤ 500 ppm en poids.

16. Procédé selon l'une quelconque des revendications 1 à 6 ou 8 à 13 pour la préparation de para-XDA présentant une teneur résiduelle en 4-méthylbenzylamine ≤ 500 ppm en poids.

17. Procédé selon l'une quelconque des revendications précédentes, le diamètre de la colonne de distillation équipée d'un soutirage latéral étant réduit dans la zone du fond par rapport au corps de colonne qui se trouve au-dessus de ce fond.
